# EUROPÄISCHE PATENTSCHRIFT

(11) **EP 0 652 019 B1**
(45) Veröffentlichungstag und Bekanntmachung des Hinweises auf die Patenterteilung: **27.01.1999**
(21) Anmeldenummer: 94250255.0
(22) Anmeldetag: 18.10.1994
(51) Int. Cl.: A61M 5/315

(54) **Injektionsspritze zum Mischen und Applizieren von Injektionssubstanzen**
Mixing and administration syringe
Seringue pour le mélange et l'administration de produits injectables

(30) Priorität: 08.11.1993 DE 4338553
(43) Veröffentlichungstag der Anmeldung: 10.05.1995
(73) Patentinhaber: Ferring Arzneimittel GmbH, D-24020 Kiel (DE)
(72) Erfinder: Blank, Norbert, D-24229 Strande (DE); Zwick, Joachim, D-24159 Kiel (DE)
(74) Vertreter: UEXKÜLL & STOLBERG

(56) Entgegenhaltungen:
- EP-A- 0 188 981
- WO-A-84/00011
- CH-A- 124 863
- DE-A- 1 961 166
- GB-A- 1 214 053

## Beschreibung

Die Erfindung betrifft eine als Doppelkammerspritze ausgebildete Injektionsspritze zum Mischen und Applizieren von Injektionssubstanzen, wobei die Injektionsspritze einen Spritzenzylinder und einen darin verschiebbaren Stempel aufweist, der mit seinem proximalen Ende aus dem Zylinder herausragt und an seinem distalen Ende mit einem Stopfen dicht an der Innenfläche des Zylinders anliegt, um ein Injektionsvolumen in dem Spritzenzylinder zu bilden.

Vielfach müssen Injektionssubstanzen vor ihrer Anwendung zusammengebracht und durchmischt werden oder sind bestimmte Lösungen zu bilden. Beispiele sind wässrige Lösungen, wässrige Suspensionen, Emulsionen O/W, W/O, Lösungen mit schwer löslichen Trockensubstanzen, ölige Lösungen, ölige Suspensionen, Lösungen mit Makrokristallen und Komprimaten. Injektionssubstanzen der obigen Art werden unmittelbar vor ihrer Anwendung zusammengebracht und gemischt bzw. gelöst. Im einfachsten Falle geschieht dies dadurch, daß die zu mischenden Substanzen in einem kleinen Gefäß angesetzt und durch Schütteln gemischt bzw. gelöst, und die durchmischte Injektionssubstanz in eine Injektionsspritze aufgezogen wird. Diese Kombination aus externem Gefäß zum Mischen der Injektionssubstanz und Injektionsspritze wird auch als Vial Spritze bezeichnet. Es ist offensichtlich, daß beim Mischen und Aufziehen von Injektionssubstanzen der obigen Art bei einer solchen Kombination aus externem Gefäß und Injektionsspritze hohe Verluste an Wirkstoff auftreten, da die in dem Gefäß angesetzte Injektionssubstanz niemals vollständig in die Injektionsspritze aufgenommen werden kann. Ferner verlangt das Mischen und Aufziehen einer Injektionssubstanz mit der Vial-Spritze einige manuelle Fertigkeiten von Ärzten und medizinischem Personal.

Ein weiteres System zum Mischen und Applizieren von Injektionssubstanzen der genannten Art besteht aus zwei üblichen Injektionsspritzen, deren Kanülen oder Kanülenansätze durch eine aufsteckbare Leitung miteinander verbunden sind. Bei diesem Zwei-Spritzen-System werden die gefüllten Spritzen mittels eines Konnektors miteinander verbunden und die Stempel der beiden Spritzen gegenläufig zueinander betätigt, so daß die Substanzen zwischen den beiden Spritzen hin- und hergepumpt werden. Die durch die Kanülenansätze bzw. die Verbindungsleitung in dem Konnektor bedingte Querschnittsverengung gegenüber den Spritzenzylindern übt beim Pumpvorgang eine erhebliche Drosselwirkung aus, woraus sich erhebliche Turbulenzen in der Strömung und dadurch bedingt eine Durchmischung der Substanzen ergibt. Auch bei dieser Lösung ist jedoch nachteilig, daß stets Wirkstoffverluste auftreten, da immer Injektionssubstanz in der nicht zum Applizieren verwendeten Spritze sowie der Verbindungsleitung zurückbleibt. Bei teuren Wirkstoffen sind die dadurch bedingten Verluste erheblich.

Der Funktion nach ähnlich wirkt die sogenannte Doppelkammerspritze, bei der die zu mischenden Substanzen von vornherein in zwei Kammern der Spritze enthalten sind. Die Doppelkammerspritzen enthält wie eine einfache Spritze einen Stempel mit einem Stopfen am Ende und darüber hinaus einen zweiten Stopfen, der zwischen dem ersten Stopfen und dem Kanülenansatz in dem Injektionsvolumen liegt und dieses in eine erste Kammer (zwischen dem Kanülenansatz und dem zweiten Stopfen) und eine zweite Kammer (zwischen dem ersten und dem zweiten Stopfen) aufteilt. Durch Niederdrücken des Stempels wird über den ersten Stopfen durch das in der zweiten Kammer befindliche Suspensionmittel sozusagen hydraulisch auch der zweite Stopfen bewegt, und zwar so weit bis er in einen Bereich des Spritzenzylinders gelangt, in dem dieser mit einer Erweiterung (Bypass) versehen ist, durch die das Suspensionsmittel den zweiten Stopfen umgeht und aus der ersten Kammer in die zweite Kammer überfließt. Die somit in der ersten Kammer zusammengebrachten Substanzen werden dann durch Aufschütteln vermischt und so die Lösung oder Suspension hergestellt. Die Durchmischung durch Aufschütteln ist jedoch nicht sehr effektiv.

Aus der DE-PS 730 362 ist eine Injektionsspritze mit mehreren in einem Zylinder hintereinanderliegenden Kammern zur Verabreichung verschiedener Injektionsmittel bekannt. Die Spritze weist einen Spritzenzylinder, einen darin verschiebbaren ersten Stempel, der an seinem distalen Ende mit einem Stopfen dicht an der Innenfläche des Zylinders anliegt, und einen verschiebbaren zweiten Stempel auf, der durch den ersten Stempel in Längsrichtung verschiebbar hindurchgeführt ist und der an seinem distalen Ende mit einem zweiten Stopfen versehen ist, so daß zwischen dem ersten und dem zweiten Stopfen eine erste Kammer und zwischen dem zweiten Stopfen und dem die Kanüle tragenden Boden des Spritzenzylinders eine zweite Kammer gebildet wird, wobei beide Kammern durch den zweiten Stopfen vollständig voneinander getrennt sind. Die Spritze ist ferner mit einer Bypass-Leitung versehen, die Öffnungen in der Seitenwand des Spritzenzylinders mit der Kanülenleitung verbindet. Die Spritze dient dazu, ein Injektionsmittel und getrennt davon vor oder nach dem Injektionsmittel ein weiteres Mittel, beispielsweise eine indifferente Lösung, zu Spritzen. Zum Gebrauch wird das Injektionsmittel in die erste Kammer und die Lösung in die zweite Kammer aufgezogen, gen, wobei der zweite Stopfen so gestellt wird, daß er die Öffnungen in der Zylinderwand zu der Bypass-Leitung hin versperrt. Dann kann durch Herabdrücken des zweiten Stopfens mit dem zweiten Stempel zunächst Lösung gespritzt werden. Sobald der zweite Stopfen um eine bestimmte Strecke hinabgedrückt ist, gibt er die Öffnungen in der Zylinderwand frei, wodurch durch die Bypass-Leitung eine Verbindung aus der ersten Kammer zur Kanüle hergestellt wird. Durch Herabdrücken des ersten Stopfens mit dem ersten Stempel kann dann das Injektionsmittel aus der ersten Kammer gespritzt werden kann. Eine Durchmischung der Mittel in der ersten und der zweiten Kammer ist mit dieser Spritzenanordnung nicht möglich.

Aus der EP 0 511 183 A1 ist eine Doppelkammerspritze bekannt mit einer ersten Kammer mit Lösungs- oder Suspensionsmittel und einer zweiten Kammer, die die zu lösende oder suspendierende Substanz enthält. In der ersten Kammer ist ein mit einem Stempel verschiebbar erster Stopfen angeordnet und in der zweiten Kammer ist ein zweiter verschiebbarer Stopfen angeordnet, der die beiden Kammern voneinander trennt. Der zweite verschiebbare Stopfen enthält ein Ventilelement, das durch hydrostatischen Druck, der durch Vorschieben des ersten Stopfens erzeugt wird, aus seinem Sitz gehoben wird und einen Durchflußweg aus der ersten in die zweite Kammer öffnet. Durch Vorschieben des ersten Stopfens kann das Lösungsmittel/Suspensionsmittel vollständig in die erste Kammer hineingedrückt werden. Dort erfolgt die Vermischung einfach durch die Vereinigung beider Mittel. Die eigentliche Injektion wird ausgeführt, indem der zweite Stopfen durch Andruck mit dem ersten Stempel vorgeschoben wird, um dadurch die erste Kammer durch eine Kanüle entleeren. Die bekannte Doppelkammerspritze sieht keine Maßnahmen vor, die eine aktive Durchmischung der Substanzen, die in der ersten Kammer vereinigt werden, ermöglichen würde.

Die den Oberbegriff von Anspruch 1 bildende WO-A-84/00011 zeigt eine Doppelkammer-Injektionsspritze mit einem Spritzenzylinder, der an seinem proximalen Ende durch einen Verschlußstopfen verschlossen ist, um in dem Spritzenzylinder zwischen dem Verschlußstopfen und dem Boden des Spritzenzylinders ein Injektionsvolumen zu bilden. Durch den Verschlußstopfen ist ein verschiebbarer Stempel geführt, der mit seinem proximalen Ende aus dem Spritzenzylinder herausragt und an seinem distalen Ende mit einem verschiebbaren Stopfen versehen ist, der zwischen dem Verschlußstopfen und dem Boden des Spritzenzylinders angeordnet ist und durch den das Injektionsvolumen in eine erste Kammer, die zwischen dem Boden des Spritzenzylinders und dem verschiebbaren Stopfen gelegen ist, und eine zweite Kammer unterteilt ist, die zwischen dem verschiebbaren Stopfen und dem Verschlußstopfen gelegen ist. Durch den verschiebbaren Stempel ist ein in Längsrichtung verschiebbarer Mischstempel geführt, der an seinem proximalen Ende aus dem verschiebbaren Stempel herausragt und an seinem distalen Ende mit einem sich in der ersten Kammer befindlichen und in dieser verschiebbaren Mischkolben versehen ist, der wenigstens eine durch die Kolbenflächen hindurchgehende Öffnung aufweist, durch die Injektionssubstanzen bei Verschieben des Mischkolbens in der ersten Kammer hindurchtreten können.

Es ist Aufgabe der vorliegenden Erfindung, eine als Doppelkammerspritze ausgebildete Injektionsspritze zum Mischen und Applizieren von Injektionssubstanzen zu schaffen, die einfach aufgebaut und handhabbar ist und die eine gute Durchmischung von mehreren Injektionssubstanzen bei minimalen Verlusten erreicht.

Zur Lösung dieser Aufgabe dient eine Injektionsspritze der eingang genannten Art mit den kennzeichnenden Merkmalen des Patentanspruchs 1. Vorteilhafte Ausgestaltungen der Erfindung sind in den Unteransprüchen aufgeführt.

Erfindungsgemäß ist durch den Stempel der Spritze ein in Längsrichtung verschiebbarer Mischstempel durchgeführt, der am proximalen Ende aus dem Stempel herausragt und an seinem distalen, im Injektionsvolumen der Spritze liegenden Ende mit einem Mischkolben versehen ist (die Begriffe proximal und distal beziehen sich auf den die Spritze ansetzenden Arzt). Der Mischkolben unterteilt das durch den Spritzenzylinder und den Stopfen des Stempels definierte Injektionsvolumen in zwei Teilbereiche. Der Mischkolben ist mit wenigstens einer hindurchgehenden Öffnung versehen, die eine Verbindung zwischen den beiden Teilbereichen des Injektionsvolumens schafft. Durch Verschieben des Mischstempels im Stempel wird der Mischkolben durch das Injektionsvolumen des Spritzenzylinders bewegt, wobei die Injektionssubtanzen durch die wenigstens eine Öffnung des Mischkolbens hindurchströmen. Der Durchtritt der Substanzen durch die drosselnde Öffnung bewirkt eine effektive Mischung. Zwischen dem Mischkolben und dem Boden des Spritzenzylinders ist ein zweiter Stopfen verschiebbar angeordnet, durch den das Injektionsvolumen in eine erste Kammer für Lösungs- oder Suspensionsmittel und in eine zweite Kammer für eine zu lösende oder zu suspendierende Substanz unterteilt ist. Der zweite Stopfen mit einem Ventilmittel versehen ist, das durch mechanische Einwirkung beim Vorschieben des Mischkolbens in Richtung auf den zweiten Stopfen zu öffnen ist, um einen Durchflußweg zwischen den beiden Kammern zu schaffen.

Durch die erfindungsgemäße Gestaltung der Injektionsspritze wird der Mischvorgang ausschließlich in dem Injektionsvolumen der Spritze durchgeführt, wodurch Verluste an Injektionssubstanzen minimiert werden, da keine weiteren externen Leitungen oder Gefäße benötigt werden, in denen Injektionssubstanzen zurückbleiben könnten.

Weiterhin ist die erfindungsgemäße Injektionsspritze sehr einfach handhabbar, da das Mischen einfach und effektiv durch Hinund Herbewegen des Mischkolbens bewirkt werden kann und die Spritze anschließend direkt zur Injektion verwendet werden kann.

Der Stempel und der erste Stopfen sind mit einer hindurchgehenden zentralen Bohrung versehen, in der der Mischstempel in Längsrichtung verschiebbar aufgenommen ist. Das in dem Spritzenzylinder liegende Injektionsvolumen wird durch an dem Mischstempel gleitfähig anliegende Dichtungslippen abgedichtet.

Die Stopfen können aus flexiblem Material bestehen und an ihrem äußeren Umfang mit umlaufenden Dichtungslippen und im Bereich der Durchbohrung an der Innenfläche mit an dem Mischstempel gleitfähig anliegenden Dichtungslippen versehen sein.

Der Mischstempel kann mit einem Wulst versehen sein, der mit einer entsprechenden Ausnehmung in der Bohrung des Stempels oder des Stopfens verrastbar ist. Dadurch kann der Mischstempel nach Abschluß des Mischvorgangs gegen Bewegung in Längsrichtung gesichert festgestellt werden.

Die proximale, dem ersten Stopfen zugewandte Oberfläche des Mischkolbens kann komplementär zu der gegenüberliegenden Oberfläche des Stopfens gestaltet. Dadurch wird bewirkt, daß Stopfen und Mischkolben in Anlage aneinander gebracht werden können, ohne das Injektionssubstanz in einem Zwischenraum zwischen ihnen zurückbleiben könnte. Vorteilhaft sind der Wulst und die Ausnehmung so zueiander angeordnet, daß die proximale Oberfläche des Mischkolbens in der eingerasteten Stellung dicht an der gegenüberliegenden Oberfläche des ersten Stopfens anliegt, so daß in der eingerasteten Stellung des Mischkolbens kein Zwischenraum zwischen Stopfen und Mischkolben verbleibt.

Der Mischstempel kann in Längsrichtung zweistückig mit einer lösbaren Kupplung gestaltet sein. Dadurch kann, nachdem durch Hin- und Herbewegen des Mischstempels die Mischung der Injektionssubstanzen durchgeführt worden ist, das aus dem proximalen Ende des Stempels herausragende Stück des Mischstempels durch Lösen der Kupplung abgenommen werden. Danach kann die Injektionsspritze von dem Benutzer wie eine übliche Injektionsspritze gehandhabt werden.

Der den Kanülenansatz tragende Boden des Spritzenzylinders kann so geformt sein, daß bei am Boden anliegendem Mischkolben ein Flüssigkeitsdurchgang von wenigstens einer Öffnung in dem Mischkolben zum Kanülenansatz verbleibt. Dies kann in einfacher Weise dadurch geschehen, daß die Grundfläche des Mischkolbens eben ausgebildet ist, während die den Kanülenansatz tragende Bodenfläche des Spritzenzylinders sich zum Kanülenansatz hin vertiefend gestaltet ist. Dadurch wird erreicht, daß, auch wenn der Mischkolben am Boden des Spritzenzylinders anliegt ein Flüssigkeitsdurchgang von der wenigstens einen Öffnung des Mischkolben zum Kanülenansatz hin verbleibt, so daß eventuell jenseits des Mischkolbens verbliebene Injektionssubstanz durch die Öffnung zum Kanülenansatz gedrückt werden kann.

Bei einer als Doppelkammerspritze ausgebildeten Injektionsspritze kann zwischen dem Stopfen und dem Mischkolben ein zweiter, frei verschiebbarer Stopfen vorgesehen, durch den der Mischstempel ebenfalls hindurchgeführt ist, so daß zwischen dem Stopfen und dem zweiten Stopfen eine Kammer für Lösungs- oder Suspensionsmittel gebildet ist. Der zweite Stopfen wird durch Herausziehen des Mischstempels bis in Anlage an den Stopfen gezogen, wodurch das Lösungs- oder Suspensionsmittel aus der Kammer herausgedrückt wird. Als Auslaß für das Lösungs- oder Suspensionsmittel dient eine durch den Mischstempel hindurchgehenden Leitung, deren Eingang bei Aufliegen des zweiten Stopfens auf dem Mischkolben in der Kammer für Lösungs- oder Suspensionsmittel liegt und und deren Ausgang am distalen Ende des Mischkolbens liegt, damit das Lösungs- oder Suspensionsmittel aus der Kammer in das zwischen Mischkolben und Kanülenansatz liegende Teilvolumen übertreten kann, worin sich die zu lösende oder suspendierende Substanz befindet. Wenn das Lösungs- oder Suspensionsmittel vollständig in das genannte Teilvolumen gebracht ist, erfolgt die Mischung mit Hilfe des Mischstempels wie zuvor. Die zu mischenden oder lösenden Substanzen werden vorteilhaft schon in der Injektionsspritze befindlich als Spritzen/Injektionsmittel-Kombination geliefert. Die Leitung ist vorteilhaft mit einem Ventil versehen, damit keine Flüssigkeit vorzeitig, d.h. vor der eigentlichen Benutzung der Spritze aus der Kammer für Lösungs- oder Suspensionsmittel austritt.

Die Erfindung wird im folgenden anhand von Ausführungsbeispielen in den Zeichnungen beschrieben, in denen:
- Figur 1: eine seitliche Querschnittsansicht einer bekannten Injektionsspritze zeigt;
- Figur 2: eine seitliche Querschnittsansicht der Spritze aus Figur 1 zeigt, wobei hier der Mischkolben nach Beendigung des Mischvorgangs an den Stopfen angezogen ist;
- Figur 3: eine seitliche Querschnittsansicht einer Ausführungsform der in Figuren 1 und 2 gezeigten Injektionsspritze als eine Doppelkammerspritze zeigt.
- Figur 4: eine seitliche Querschnittsansicht einer erfindungsgemäßen Doppelkammerspritze zeigt;
- Figur 4a: eine perspektivische Detailansicht des Mischkolbens der Doppelkammerspritze aus Figur 4 zeigt; und
- Figur 5: eine Querschnittsansicht der Doppelkammerspritze aus Figur 4 in einer späteren Benutzungsphase beim Durchmischen zeigt.

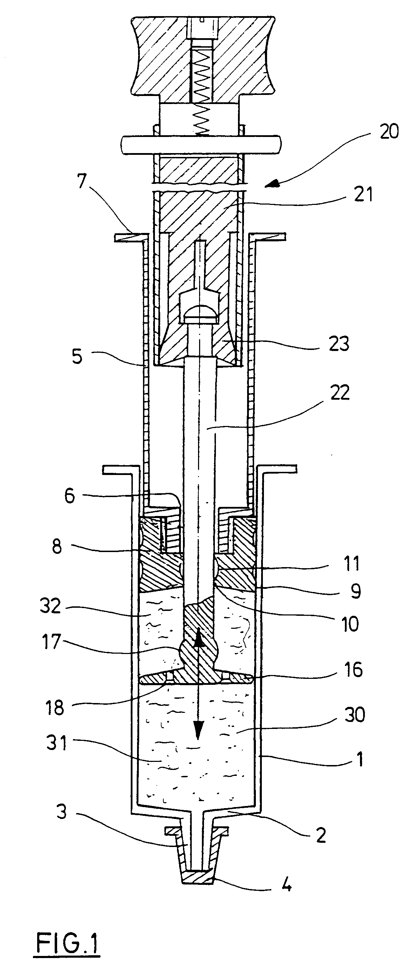

Die bekannte Injektionsspritze in Figur 1 weist einen Spritzenzylinder 1 auf, in dem verschiebbar ein Stempel 5 geführt ist. Der verschiebbare Stempel 5 ist an seinem in dem Spritzenzylinder 1 liegenden Ende mit einem Stopfen 8 versehen, der dicht an der Innenfläche des Spritzenzylinders 1 anliegt. In der gezeigten Ausführungsform ist der Stopfen 8 aus nachgiebigem Material, beispielsweise Gummi, hergestellt und weist drei am äußeren Umfang umlaufende Dichtungslippen 9 auf. Durch den Spritzenzylinder 1 und den Stopfen 8 wird ein Injektionsvolumen 30 definiert. Am distalen Ende des Spritzenzylinders 1 ist zentrisch ein Kanülenansatz 3 angeordnet, der mit einer Kappe 4 verschließbar ist.

Der Stempel 5 weist an seinem proximalen Ende 7 einen flanschartigen Anlagegriff auf, an dem angreifend der Benutzer die Injektionsspritze aufziehen und gegen den andrückend die in dem Injektionsvolumen 30 befindliche Substanz appliziert werden kann. Der Stempel 5 ist mit einer hindurchgehenden, zentralen Bohrung 6 versehen, die sich durch den Stopfen 8 hindurchgehend in das Injektionsvolumen 30 öffnet. In der hindurchgehenden Bohrung 6 ist gleitfähig ein verschiebbarer Mischstempel 20 geführt. Die hindurchgehende Bohrung 6 wird durch in der Durchbohrung des Stopfens 8 vorgesehene innere Dichtungslippen 10 gegen das Injektionsvolumen 30 abgedichtet.

Der verschiebbare Mischstempel 20 ist an seinem distalen, in dem Injektionsvolumen 30 liegenden Ende mit einem Mischkolben 16 versehen. Der in dem Injektionsvolumen 30 liegende Mischkolben 16 unterteilt dieses in ein erstes Teilvolumen 31 und ein zweites Teilvolumen 32. Die beiden Teilvolumina 31 und 32 stehen durch wenigstens eine in dem Mischkolben 20 gebildete Öffnung 18 miteinander in Verbindung. Der Begriff Öffnung ist dabei allgemein so zu verstehen, daß ein Flüssigkeitsstrom durch oder an dem Mischkolben 16 vorbei aus dem ersten Teilvolumen 31 in das zweite Teilvolumen 32 und umgekehrt möglich ist. Dies kann beispielsweise auch dadurch erreicht werden, daß der Außendurchmesser des Mischkolbens 16 kleiner als der Innendurchmesser des Spritzenzylinders 1 ist oder der äußere Umfang oval oder sternförmig gestaltet ist oder der Mischkolben 16 mit Schrägbohrungen/Schrägschlitzen versehen ist. In der dargestellten bevorzugten Ausführungsform liegt der äußere Umfang des Mischkolbens 16 jedoch an der Innenfläche des Spritzenzylinders 1 an, und es sind zwei hindurchgehende Öffnungen 18 durch den Mischkolben 16 gebildet.

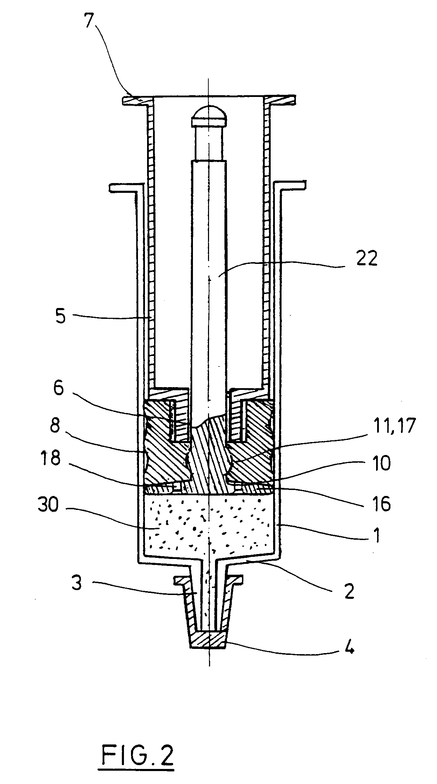

In Benutzung werden die zu durchmischenden Injektionssubstanzen wie üblich in die Injektionsspritze aufgezogen, wobei die Spritze bei Lieferung in der Regel die zu lösende oder in Suspension zu bringende Substanz in ihrem Injektionsvolumen bereits enthält. Dieser Zustand ist in Figur 2 gezeigt, wobei sich die in Lösung oder Suspension zu bringende Substanz in dem Injektionsvolumen 30 befindet und die Spritze als Spritze-Wirkstoff-Kombination konfektioniert dem Anwender zur Verfügung gestellt wird. Dann braucht nur noch das Lösungs- oder Suspensionsmittel aufgezogen zu werden. Der Mischvorgang wird anschließend dadurch bewirkt, daß, bei verschlossenem Kanülenansatz 3 und festgehaltenem Stempel 5, der Mischkolben 16 durch Hin- und Herschieben des Mischstempels 20 im Injektionsvolumen 30 hin- und herbewegt wird. Dabei müssen die zu mischenden Injektionssubstanzen durch die Öffnung 18 strömen, da sich die Volumina des ersten Teilvolumens 31 und des zweiten Teilvolumens 32 mit Verschieben des Mischkolbens ständig ändern. Durch die Strömung der Injektionssubstanzen durch die Öffnung 18 tritt eine durchmischende Wirkung auf, da es im Bereich der Öffnungen zu erheblich erhöhten Strömungsgeschwindigkeiten und gegebenenfalls Turbulenzen in der Strömung kommt, die zu einer Durchmischung der Substanzen führen.

Nachdem die Injektionssubstanzen durch mehrfaches Hin- und Herschieben des Mischstempels 20 durchmischt sind, kann die Injektionsspritze wie eine übliche Injektionsspritze verwendet werden. Der Mischstempel 20 ist so gestaltet, daß er in Längsrichtung aus zwei trennbaren Teilen 21 und 22 besteht, die durch eine Kupplung 23 miteinander verbunden sind. Nachdem der Mischvorgang abgeschlossen ist, kann das proximale Teil 21 des Mischstempels 20 durch Betätigung der Kupplung 23 gelöst und abgenommen werden. Figur 2 zeigt die Injektionsspritze, bei der das proximale Teil 21 (Figur 1) des Mischstempels 20 nicht auf das distale Teil 22 aufgesetzt ist. In dieser Konfiguration ist die Spritze besonders einfach durch Betätigen des Stempels 5 an seinem am proximalen Ende 7 vorgesehen flanschartigen Anlagegriff zu betätigen, ohne das der Mischstempel 20 durch das herausragende distale Teil 21 das Applizieren der Injektion stört.

Der Mischstempel 20 ist nahe an seinem distalen Ende in einem Bereich vor dem Mischkolben 16 mit einem Wulst 17 gestaltet, der geeignet ist, mit einer zwischen den Dichtungslippen 10 am Stopfen 8 liegenden Ausnehmung 11 zu verrasten, um so den Mischstempel 20 in dem Stempel 5 festzustellen. Dieses Feststellen des Mischstempels kann nach erfolgter Durchmischung der Injektionssubstanzen durchgeführt werden, wonach das proximale Teil 21 des Mischstempels 20 durch Lösen der Kupplung 23 entfernt und die Spritze zum Applizieren der durchmischten Injektionssubstanzen wie eine übliche Injektionsspritze verwendet werden kann. Figur 2 zeigt diesen Zustand, in dem der Mischkolben 16 an den Stopfen 8 angezogen und das distale Teil 22 des Mischstempels 20 mit seinem Wulst 17 in der Ausnehmung 11 in der Durchbohrung des Stopfens 8 eingerastet ist.

Der Mischkolben 16 ist an seiner oberen, dem Stopfen 8 zugewandten Seite komplementär zu diesem geformt, indem der Mischkolben 16 mit leicht abfallender Oberfläche und der Stopfen 8 ebenfalls mit einer leicht abfallenden Oberfläche gestaltet sind, so daß der Stopfen 8 und der Mischkolben 16 in dichte Anlage miteinander kommen können, wenn der Mischstempel 20 nach oben gegen den Stempel 5 gezogen oder der Stempel 5 nach unten gegen den Mischstempel 20 gedrückt wird. Durch die komplementär geformten Oberflächen des Stopfens 8 und des Mischkolbens 16 wird bewirkt, daß keine Injektionssubstanzen in einem etwaigen Zwischenraum zwischen beiden zurückbleiben kann. Die oben erwähnte Einrastung des Mischkolbens 16 ist so ausgelegt, daß der Mischkolben 16 in der eingerasteten Stellung dicht an dem Stopfen 8 anliegt.

Der Spritzenzylinder 1 ist an seinem den Kanülenansatz 3 tragenden Ende mit einem sich zu dem Kanülenansatz hin leicht absenkenden Boden 2 versehen, während die gegenüberliegende Oberfläche des Mischkolbens 16 eben ist. Diese Gestaltung stellt sicher, daß keine dichte Anlage des Mischkolbens 16 auf den Boden 2 erfolgen kann, in der eine Durchflußmöglichkeit durch die Öffnungen 18 zum Kanülenansatz 3 versperrt wäre. Auf diese Weise ist gewährleistet, daß, auch wenn sich beim Applizieren noch Injektionssubstanz in dem zweiten Teilvolumen 32 befindet, d.h. der Mischkolben 16 nicht ganz an den Stopfen 8 herangezogen ist, in dem zweiten Teilvolumen 32 verbliebene Injektionssubstanzen beim Niederdrücken des Stempels 5 durch die Öffnung 18 des Mischkolbens 16 zum Kanülenansatz fließen kann, so daß keine Reste von Injektionssubstanzen in dem zweiten Teilvolumen 32 verbleiben können.

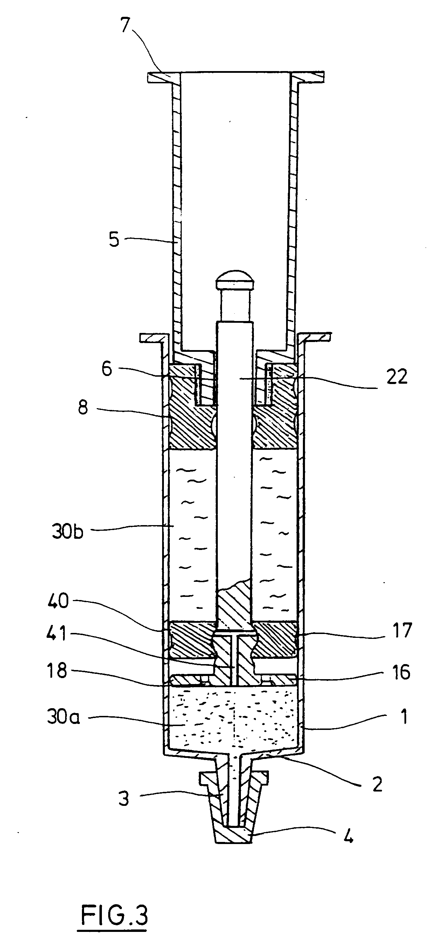

In Figur 3 ist eine als Doppelkammerspritze ausgebildete Injektionsspritze. Bei dieser Spritze ist zwischen dem Stopfen 8 und dem Mischkolben 16 ein zweiter Stopfen 40 vorgesehen, durch den der Mischstempel 20 mit seinem distalen Teil 22 hindurchgeführt ist. Zwischen dem ersten Stopfen 8 und dem zweiten Stopfen 40 ist eine Kammer 30b für Lösungs- und Suspensionsmittel gebildet, während zwischen dem zweiten Stopfen 40 und dem Boden 2 des Spritzenzylinders eine Kammer 30a für zu lösende oder suspendierende Substanzen (z.B. pulverige Substanzen, Mikrokapseln etc.) liegt. Die Doppelkammerspritze kann vorteilhaft mit Lösungs- oder Suspensionsmittel in der Kammer 30b und mit dem zu lösenden Wirkstoff in der Kammer 30a konfektioniert und in dieser Kombination ausgeliefert werden. Zur Benutzung wird dann das proximale Teil 21 (siehe Figur 1) des Mischstempels 20 auf das distale Teil 22 aufgesetzt und der Mischstempel 20 dann in Richtung aus dem Spritzenzylinder herausgezogen, während der Stempel 5 gleichzeitig festgehalten wird. Dadurch baut sich in der Kammer 30b mit Lösungs- oder Suspensionsmittel genügend Druck auf, so daß sich der zweite Stopfen 40 aus der Arretierung an dem Wulst 17 des Mischstempels löst, wobei sich die seitlichen Öffnungen der Leitung 41 zu der Kammer 30b hin öffnen und das Lösungs- oder Suspensionsmittel aus der Kammer 30b durch die Leitung 41 in die Kammer 30a übertritt und dort mit der zu lösenden Substanz zusammengebracht wird. Wenn durch Herausziehen des Mischstempels das gesamte Lösungs- oder Suspensionsmittel aus der Kammer 30b in die Kammer 30a übergetreten ist kann dort der Mischvorgang durch Hin- und Herbewegen des Mischstempels wie oben beschrieben bewirkt werden.

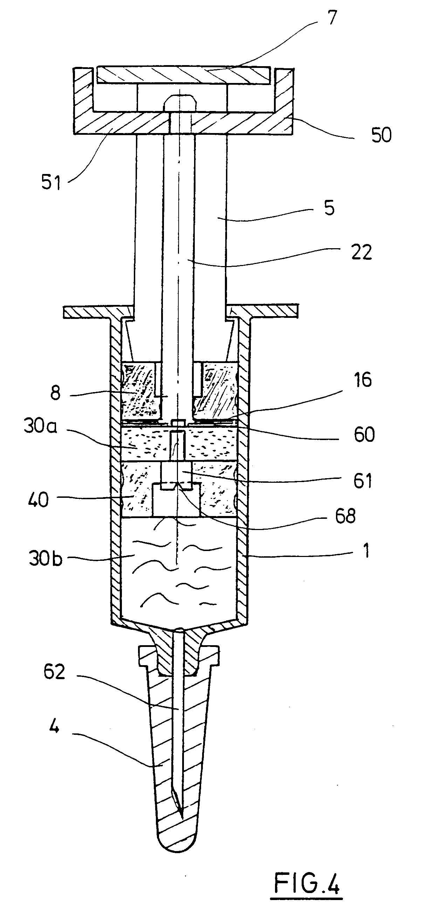

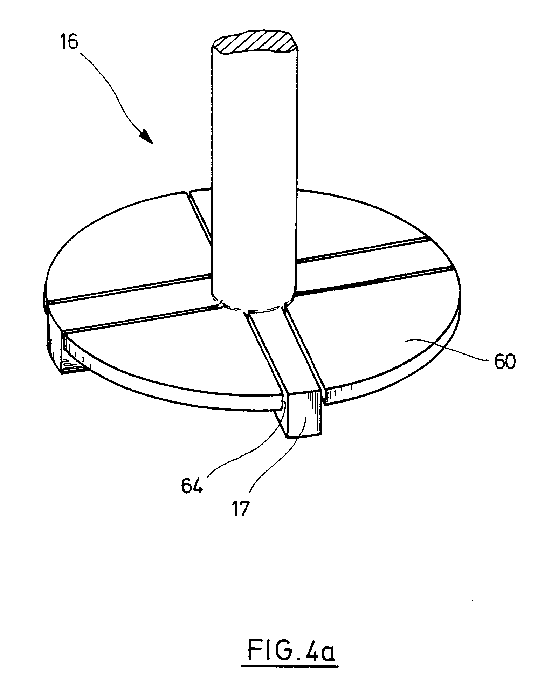

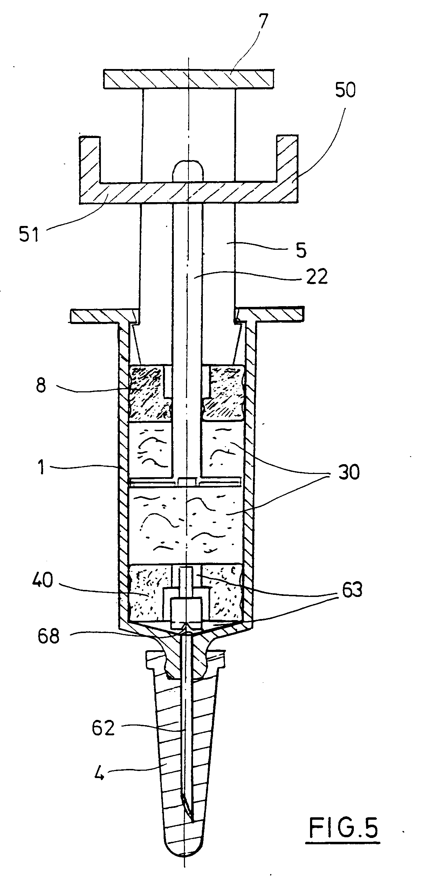

In den Figuren 4, 4a und 5 ist eine erfindungsgemäße, als Doppelkammerspritze ausgebildeten Injektionsspritze gezeigt. Figur 4 zeigt die Spritze in einem vorbereiteten Zustand gefüllt mit zu lösender/suspendierender Substanz und Lösungsmittel/Suspensionsmittel, wobei die Spritze bereits beim Hersteller derart vorbereitet und zu den Benutzern ausgeliefert werden kann. In dieser Ausführungsform liegt die Kammer 30a für die zu lösende oder suspendierende Substanz im oberen Teil, während die Kammer 30b mit Lösungs- oder Suspensionsmittel in dem der Kanüle 62 zugewandten Teil des Injektionsvolumens liegt. Die Kammern 30a und 30b sind in dem in Figur 4 gezeigten Zustand durch den zweiten Stopfen 40 voneinander getrennt, der verschiebbar in dem Injektionsvolumen des Spritzenzylinders 1 sitzt. Der zweite Stopfen 40 weist eine Bohrung auf, in die ein Kegelstift 61 eingesetzt ist, der einen dichten Verschluß bildet. Der Kegelstift 61 kann durch Andruck auf sein von der Kanüle 62 abgewandtes Ende aus dem Sitz in der Bohrung des zweiten Stopfens 41 vorgeschoben werden.

Die Gestaltung des Mischkammerkolbens 16 ist am besten in Figur 4a zu erkennen. Die durch die Kolbenfläche hindurchgehenden Öffnungen sind in diesem Fall als Schlitze 64 ausgebildet, wobei jeweils ein Paar von Schlitzen zwischen sich einen Steg 17 definiert. Es kann eine Mehrzahl solcher Schlitzpaare vorgesehen sein, beispielsweise vier Schlitzpaare, so daß vier Stege 17 in kreuzförmiger Anordnung gebildet werden. Zwischen benachbarten Stegen 17 sind in ihrer Dicke reduzierte flexible Bereiche 60 vorgesehen. Das Material des Kolbens 16, vorzugsweise ein Kunststoff, und die Dicke der flexiblen Bereiche 60 sind so gewählt, daß die flexiblen Bereiche 60 bei Andruck auf darunterliegendes Material sich nach oben biegen können.

Um die in Figur 4 gezeigte Spritze in Benutzung zu nehmen, wird sie wie folgt betätigt. Der Benutzer bewegt den Mischstempel 22, indem er einen außen über den Stempel 5 hervorstehenden Griff 50 faßt, der beispielsweise außen ringförmig ist und über Streben 51 mit dem Mischstempel 22 verbunden ist, wobei die Streben 51 durch Schlitze in dem Stempel 5 hindurchgeführt sind. Der Griff 50 und damit der Mischstempel 22 werden nach unten gedrückt. Dadurch wird der Mischkolben 16 nach unten in die Kammer 30a mit der zu lösenden Substanz hineingedrückt, wobei die flexiblen Bereiche 60 (siehe Figur 4a) dem Gegendruck durch die zu lösende Substanz ausweichen, indem sie nach oben durchbiegen. Dadurch kann sich der Mischkolben dem relativ fest in dem Spritzenzylinder 1 sitzenden zweiten Stopfen 40 annähern und in Anlage an einen Vorsprung des Kegelstiftes 61 kommen. Durch weiteren Andruck löst der an dem Vorsprung des Kegelstiftes 61 anliegende Mischkolben 16 den Kegelstift 61 nach unten aus seinem Sitz in dem Stopfen 40 heraus. Der genannte Vorsprung des Kegelstiftes ist seitlich an zwei Seiten abgeflacht. Nach dem Verschieben entstehen so zwei Strömungskanäle im Stopfen 40, so daß eine Flüssigkeitsverbindung zwischen den Kammern 30a und 30b durch die Bohrung des Stopfens 40 hergestellt wird. Anschließend kann Flüssigkeit aus der Kammer 30b durch die Bohrung in dem Stopfen 40 und die Spalte 64 in dem Mischkolben 16 in die Kammer 30a hindurchtreten. Auf diese Weise kann der Stopfen 40 mit Hilfe des Mischstempels 22 zu dem an der Seite der Kanüle 62 liegenden Ende des Spritzenzylinders 1 hinuntergedrückt werden.

Die genannte heruntergedrückte Lage des Stopfens 40 ist in Figur 5 gezeigt. In dieser Stellung kann wie in den vorherigen Ausführungsbeispielen eine gute Durchmischung bewirkt werden, indem der Mischkolben 16 durch Hin- und Herbewegen des Mischstempels 22 hin- und herbewegt wird, wodurch die zu lösende Substanz mit dem Lösungsmittel beim Durchtritt durch die Spalte 64 gut vermischt wird.

Der Kegelstift ist an seiner Bodenfläche 2 des Spritzenzylinders 1 zugewandten Seite so ausgebildet, daß er bei Anlage an der Bodenfläche 2 einen Durchflußweg in die Kanüle 62 freiläßt. Dies kann z.B. dadurch geschehen, daß an der der Bodenfläche 2 zugewandten Seite des Kegelstiftes 61 Nuten 68 vorgesehen werden.

Anstelle des in dem Stopfen 40 sitzenden Kegelstiftes 61 kann jede Art von Ventilmittel verwendet werden, das geeignet ist, durch Krafteinwirkung durch den Mischkolben 16 eine Durchflußverbindung 63 durch den Stopfen 40 freizugeben. Als weitere Ventilmittel, die durch mechanische Einwirkung des sich vorschiebenden Mischkolbens 16 zu öffnen sind, kommt z.B. eine dünne Materialschicht in Betracht, die eine Bohrung in dem zweiten Stopfen 40 verschließt und durch einen Dorn oder irgendein anderes Mittel an dem Mischkolben 16 geöffnet werden kann, um so einen Durchflußweg durch den zweiten Stopfen 40 zu schaffen.

## Patentansprüche

1. Als Doppelkammerspritze ausgebildete Injektionsspritze zum Mischen und Applizieren von Injektionssubstanzen, mit
- einem Spritzenzylinder (1),
- einem darin verschiebbaren Stempel (5), der mit seinem proximalen Ende aus dem Zylinder (1) herausragt und an seinem distalen Ende mit einem ersten Stopfen (8) dicht an der Innenfläche des Zylinders (1) anliegt, um ein Injektionsvolumen (30) in dem Spritzenzylinder (1) zu bilden,
- einem Mischstempel (22), der in Längsrichtung verschiebbar durch den Stempel (5) hindurchgeführt ist, mit seinem proximalen Ende aus dem Stempel (5) herausragt und an seinem distalen Ende mit einem Mischkolben (16) versehen ist, der wenigstens eine durch die Kolbenfläche hindurchgehende Öffnung (18) aufweist, durch die Injektionssubstanzen hindurchtreten können, wenn der Mischkolben (16) im Injektionsvolumen (30) verschoben wird,
**dadurch gekennzeichnet,** daß
- ein zweiter Stopfen (40) zwischen dem Mischkolben (16) und dem Boden (2) des Spritzenzylinders (1) verschiebbar angeordnet ist, durch den das Injektionsvolumen (30) in eine erste Kammer (30b) für Lösungs- oder Suspensionsmittel und in eine zweite Kammer (30a) für eine zu lösende oder zu suspendierende Substanz unterteilt ist, und
- der zweite Stopfen (40) mit einem Ventilmittel (61) versehen ist, das durch mechanische Einwirkung beim Vorschieben des Mischkolbens (16) in Richtung auf den zweiten Stopfen (40) zu öffnen ist, um einen Durchflußweg zwischen den beiden Kammern (30a, 30b) zu schaffen.

2. Injektionsspritze nach Anspruch 1, **dadurch gekennzeichnet**, daß das Ventilmittel (61) durch einen in einer Bohrung des zweiten Stopfens (40) sitzenden Kegelstift gebildet wird, der bei Andruck auf ein dem Mischkolben (16) zugewandtes Ende aus seinem Sitz in der Bohrung des Stopfens (40) lösbar ist.

3. Injektionsspritze nach Anspruch 2, **dadurch gekennzeichnet**, daß der Kegelstift (61) an seiner dem Boden (2) des Spritzenzylinders (1) zugewandten Seite mit Nuten (68) versehen ist, die einen Durchflußweg zwischen Boden (2) und Kegelstift (61) freihalten.

4. Injektionsspritze nach Anspruch 1, **dadurch gekennzeichnet**, daß der Mischkolben (16) flexible Bereiche (60) aufweist, die einem Andruck senkrecht zur Mischkolbenfläche durch Durchbiegen teilweise ausweichen können.

## Claims

1. Injection syringe, constructed as a double-chamber syringe, for mixing and administration of substances for injection, having
- a syringe barrel (1),
- a plunger (5) which can be displaced therein, projects out of the barrel (1) with its proximal end and lies tightly against the inner surface of the barrel (1) with a first stopper (8) at its distal end, to form an injection volume (30) in the syringe barrel (1),
- a mixing plunger (22) which is passed through the plunger (5) such that it can be displaced in the longitudinal direction, projects out of the plunger (5) with its proximal end and is provided, on its distal end, with a mixing piston (16) which has at least one opening (18) which passes through the piston surface and through which substance for injection can pass when the mixing piston (16) is displaced in the injection volume (30),
characterized in that
- a second stopper (40) is arranged between the mixing piston (16) and the base (2) of the injection barrel (1) in a manner such that it can be displaced, by which the injection volume (30) is divided into a first chamber (30b) for solvent or suspending agent and into a second chamber (30a) for a substance to be dissolved or to be suspended, and
- the second stopper (40) is provided with a valve means (61) which is to be opened by mechanical action when the mixing piston (16) is pushed forwards in the direction of the second stopper (40), to provide a flow-through path between the two chambers (30a, 30b).

2. Injection syringe according to claim 1, characterized in that the valve means (61) is formed by a tapered pin which sits in a bore of the second stopper (40) and can be detached from its seat in the bore of the stopper (40) under contact pressure on one end facing the mixing piston (16).

3. Injection syringe according to claim 2, characterized in that the tapered pin (61) is provided, on its side facing the base (2) of the injection barrel (1), with grooves (68) which keep a flow-through path between the base (2) and tapered pin (61) free.

4. Injection syringe according to claim 1, characterized in that the mixing piston (16) has flexible regions (60) which can partly evade a contact pressure perpendicular to the mixing piston surface by bending.

## Revendications

1. Seringue d'injection réalisée sous forme de seringue à chambre double pour le mélange et l'administration de matières d'injection, comportant
- un cylindre de seringue (1),
- un piston (5) déplaçable dans celui-ci qui dépasse avec son extrémité proximale du cylindre (1) et qui s'applique, à son extrémité distale, avec un premier bouchon (8), d'une manière étanche à la surface intérieure du cylindre (1), pour former un volume d'injection (30) dans le cylindre de seringue (1),
- un piston de mélange (22) qui est guidé d'une manière déplaçable dans la direction longitudinale à travers le piston (5), qui dépasse avec son extrémité proximale du piston (5) et qui est pourvu à son extrémité distale d'un piston de mélange (16) qui présente au moins une ouverture (18) passant à travers la surface de piston, à travers laquelle les matières d'injection peuvent passer lorsque le piston de mélange (16) est déplacé dans le volume d'injection (30),
caractérisée en ce qu'un deuxième bouchon (40) est disposé de manière déplaçable entre le piston de mélange (16) et le fond (2) du cylindre de seringue (1), par lequel le volume d'injection (30) est divisé en une première chambre (30b) pour un solvant ou un agent de suspension et en une deuxième chambre (30a) pour une matière à dissoudre ou à mettre en suspension, et
- en ce que le deuxième bouchon (40) est pourvu d'un moyen de vanne (61) qui peut être ouvert sous l'action mécanique lors de la poussée en avant du piston de mélange (16) en direction du deuxième bouchon (40) pour créer un trajet d'écoulement entre les deux chambres (30a, 30b).

2. Seringue d'injection selon la revendication 1, caractérisée en ce que le moyen de vanne (61) est formé par une tige conique située dans un perçage du deuxième bouchon (40) qui, lors d'une pression exercée sur une extrémité orientée vers le piston de mélange (16), peut être sortie de son siège dans le perçage du bouchon (40).

3. Seringue d'injection selon la revendication 2, caractérisée en ce que la tige conique (61) est pourvue à son côté orienté vers le fond (2) du cylindre de seringue (1) de rainures (68) qui ménagent un trajet d'écoulement entre le fond (2) et la tige conique (61).

4. Seringue d'injection selon la revendication 1, caractérisée en ce que le piston de mélange (16) présente des zones flexibles (60) qui peuvent s'écarter partiellement d'une pression appliquée perpendiculairement à la surface du piston de mélange par un fléchissement.
